(19)

Europäisches
Patentamt
European
Patent Office
Office européen
des brevets

(11) **EP 4 368 115 A1**

(12) **EUROPEAN PATENT APPLICATION**

(43) Date of publication:
**15.05.2024 Bulletin 2024/20**

(21) Application number: **22206568.2**

(22) Date of filing: **10.11.2022**

(51) International Patent Classification (IPC):
**A61B 8/08** *(2006.01)* **A61B 8/00** *(2006.01)*

(52) Cooperative Patent Classification (CPC):
**A61B 8/0866; A61B 8/0883; A61B 8/4227;
A61B 8/4236; A61B 8/4416; A61B 8/4477;
A61B 8/4494; A61B 8/488; A61B 8/5269;
A61B 8/5276; A61B 8/5292; A61B 8/582**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **LASHINA, Tatiana Aleksandrovna
Eindhoven (NL)**
• **RABOTTI, Chiara
Eindhoven (NL)**
• **HAMELMANN, Paul Christoph
5656AG Eindhoven (NL)**

(74) Representative: **Philips Intellectual Property &
Standards
High Tech Campus 52
5656 AG Eindhoven (NL)**

(54) **CARDIOTOCOGRAPHIC SCANNING SESSION DURATION**

(57) Proposed concepts aim to provide schemes, solutions, concepts, designs, methods and systems pertaining to predicting a remaining duration of a cardiotocograph (CTG) scanning session of a subject. In particular, CTG data and movement data of the subject during the scanning session are obtained. This data is then analysed to identify artifacts in the CTG data associated with movement of the subject. From the CTG data, movement data and artifacts, a remaining duration of the CTG scanning session can be predicted. In this way, despite the potential for signal drop-outs during the scanning session, acquisition of a sufficient amount of CTG data during the scanning session may be assured.

FIG. 1

EP 4 368 115 A1

**Description**

FIELD OF THE INVENTION

The present invention relates to predicting a cardiotocographic (CTG) scanning session duration.

BACKGROUND OF THE INVENTION

Typically, those with a risky or complicated pregnancy (e.g. those who have had an episode of preterm labor, or having gestational hypertension disorder), are typically monitored at a hospital, inpatient or outpatient. A key aspect of this is monitoring of the fetal well-being with cardiotocographic (CTG) fetal monitoring.

CTG measurements are traditionally performed using a CTG fetal monitor with an ultrasound Doppler transducer for detecting fetal heart rate (fHR) and a tocodynamometer for measuring uterine activity (i.e. maternal contractions). Recently, several electrophysiology-based CTG (eCTG) devices have been commercially released and are already used by clinicians. eCTG offers many advantages over ultrasound Doppler based CTG, such as a higher fHR resolution and simpler operation.

One of the challenges for eCTG is that it is susceptible to noise, meaning that the fHR may be obscured. Specifically, the fHR signal amplitude is much lower than that of the maternal heart rate, with uterine activity and other maternal movement also obscuring the fHR signal. Via signal processing techniques, like blind source separation, fHR signal gets extracted and distinguished from these other signals, including maternal heart rate and uterine activity. Accordingly, fHR signal drop-out is a common problem for CTG measurements. fHR signals acquired from ultrasound Doppler based CTG may also not be ideal (e.g. due to improper transducer placement, fetal movement, etc.).

Healthcare professionals usually assess the acquired signal during the scanning session in order to determine the duration of the scanning session sufficient to obtain data sufficient for interpretation. This duration may be particularly dependent on the number of fHR signal dropouts, and an overall quality of the data, but also on the status of the fetus, since in order to be able to clinically conclude on the fetal well-being a fetus should not be in a deep sleep state. However, this process is particularly labor intensive, and not always accurate.

Furthermore, the assessment of a session duration is a particular issue for home-based CTG solutions, where untrained users conduct the scan and subsequently transfer the acquired data to a healthcare professional. It may be the case that a healthcare professional is not present, even remotely, for the assessment of the data at the time data gets collected. Thus, the acquisition of sufficient amounts of quality fHR data may be left to chance.

SUMMARY OF THE INVENTION

The invention is defined by the claims. Proposed concepts aim to provide schemes, solutions, concepts, designs, methods and systems pertaining to predicting a remaining duration of a cardiotocographic (CTG) scanning session of a subject. In particular, CTG data and movement data of the subject during the scanning session are obtained. This data is then analysed to identify artifacts in the CTG data associated with movement of the subject. From the CTG data, movement data and artifacts, a remaining duration of the CTG scanning session can be predicted. In this way, despite the potential for signal drop-outs during the scanning session, acquisition of a sufficient amount of CTG data during the scanning session may be assured.

According to examples in accordance with an aspect of the invention, there is provided a method for predicting a remaining duration of a cardiotocography (CTG) scanning session of a subject, the method comprising:

obtaining CTG data associated with the subject during a CTG scanning session;
obtaining movement data describing a movement of the subject during the CTG scanning session;
identifying artifacts in the CTG data associated with the movement of the subject based on the CTG data and the movement data; and
analyzing the CTG data, the movement data and the artifacts to predict a remaining duration of the CTG scanning session.

In this way, an amount of time (i.e. a remaining duration) needed in order to obtain sufficient CTG data from the CTG scanning session may be predicted. Sufficient data may be considered enough data such that the CTG data may be useful for diagnostic purposes. For example, there must be adequate amounts of uninterrupted fetal heart rate (fHR) data, such that the fHR may be properly analyzed by a healthcare professional.

Typically, the amount of remaining time required for completion of a session is determined by a healthcare professional, who looks at the CTG data already acquired and makes a judgement as to how much more is required. This process is labor intensive, may be inaccurate, and may not be available (i.e. in a self/home CTG scanning session). Of course, one solution would be to conduct the scanning session for much longer than required. However, this wastes the time of the pregnant patient, the time of the person performing the scan, and reduces access for other pregnant patients needing a CTG scan. Therefore, there exists a need for an automatic, accurate, way to predicting a remaining duration of the scanning session.

Accordingly, it has been realized that CTG data and movement data from the scanning session may be leveraged in order to determine the (required) remaining duration. Indeed, by identifying artifacts within the CTG data (alongside the motion and CTG data), a number and length of drop-outs of the CTG signal may be detected. Thus, a usability/signal coverage of the CTG data can be determined, from which a (predicted) remaining duration is derived.

Motion of the subject (i.e. the pregnant patient) often results in a reduced quality of the CTG data. This is often because such movement increases the amount of noise detected by the CTG scanner/sensor, thus reducing a signal-to-noise ratio. In cases when a pregnant patient is moving a lot it would help to instruct the patient that they should be at rest during the measurements to help improve the signal quality.

Fetal movement data is also relevant for determining the session duration, since it is a clinically relevant parameter, and it is indicative of whether the fetus is in a deep sleep state or not. Typically, in order to be able to interpret the CTG the fetus should not be in a deep sleep state. In case the fetus is in a deep sleep state (which could be determined from the fHR pattern and the presence of fetal movements) the CTG session should be prolonged to capture the period when the fetus is not in deep sleep. Accordingly, analysing the maternal and fetal movement data, alongside CTG data and artifacts therein, enables the prediction of a remaining duration required for adequate signal coverage.

In some embodiments, the method may further comprise processing the CTG data to extract feature data indicative of a usability of the CTG data for diagnosis. In this case, predicting the remaining duration is further based on the extracted feature data.

Indeed, a key determining factor of how much longer the scanning session needs to be is the quality of already acquired CTG data. Features of the CTG data indicative of the usability of such data is therefore important to assess in order to accurately determine how much longer is required for the usability of the obtained CTG data for diagnosis to be satisfactory.

Also, the feature data may comprise at least one of a fetal heart rate measurement, a uterine activity measurement, and a fetal wake status. Specifically, the fetal heart rate measurement may comprise heart rate features including a fetal heart rate variability, a fetal heart rate baseline, fetal heart rate accelerations, and fetal heart rate decelerations.

All of the above factors may be useful for determining the usability of the obtained CTG data. The fHR measurement (including the features therein) is often the most important signal to acquire by CTG, and therefore assessing this data is the most important factor upon which remaining session time is dependent. Uterine activity may also be extracted from the CTG data. A fetal sleep status may also positively impact the usability/quality of the data (i.e. CTG scanning sessions are usually conducted when the fetus is not in a deep sleep state).

Thus, by accounting for this feature data, a more accurate prediction of remaining CTG scanning session duration may be performed.

In some embodiments, the method may further comprise obtaining previous CTG data associated with the subject during at least one previous CTG scanning session. Predicting the remaining duration may then be further based on the historic CTG data.

In this way, an accuracy of prediction of the remaining duration may be improved. Indeed, there may be various clues obtained from past CTG scanning sessions of the subject. This may include an average overall scanning session time, predictable patterns in useful CTG data, down-time periods in past acquisitions, etc. As a result, if the current CTG scanning session appears similar (or divergent) to a previous CTG scanning session of the subject, the remaining duration may be predicted.

Furthermore, the method may also comprise obtaining physiological data describing one or more characteristics of the subject, and wherein predicting the remaining duration is further based on the physiological data. The physiological data may comprise at least one of an age, a gestational age, a height, a weight, medical conditions and a medical history, including obstetric history.

Certain demographic characteristics of the subject may also provide information useful for predicting the remaining duration of the session. For example, those with a lower gestational age may need a longer overall scanning session duration due to higher complexity of interpreting the fetal heart rate pattern in a less mature fetus. Thus, by taking physiological data into account, a more accurate predicted remaining duration may be provided.

Specifically, the method may further comprise matching the subject with a plurality of similar subjects based on the physiological data of the subject and physiological data of the plurality of other subjects, wherein each of the plurality of similar subjects are associated with historical CTG scanning session duration data. The predicted remaining duration may thus be further based on the historical CTG scanning session duration data.

Utilizing CTG data of scanning sessions of other subjects may also provide information useful for accurately predicting a remaining scanning duration. Indeed, subjects with similar physiological characteristics (e.g. age, gestational age, weight, etc.) may require roughly similar scanning session durations. Therefore, by taking the scanning session durations of other, similar, subjects into account, the CTG, motion and artifact data may be contextualized in order to produce a more accurate remaining duration for the scanning session.

Matching the subject may comprise obtaining physiological data of each of the plurality of other subjects, clustering the plurality of other subjects based on the physiological data to classify similar subjects into one of a plurality of classes, and matching the subject to one of the plurality of classes based on the physiological data of the subject.

One method for identifying similar subjects to the pregnant patient in question is to cluster groups of similar subjects, and then identify which group the pregnant patient is most similar to. Accordingly, more relevant CTG data of other subjects may be analyzed, improving an accuracy of the predicted remaining duration.

In some embodiments, the method may also comprise obtaining contextual information describing a context of the CTG scanning session. Predicting the remaining duration may be further based on the contextual information. In particular, the contextual information may comprise at least one of a time of day, a location, an ambient temperature and a device used to obtain the CTG data.

Once again, contextual data (e.g. environmental data and time-related data) may also provide information useful for contextualizing obtained CTG data, as well as being indicative of an overall remaining duration itself. For example, it may be known that the fetus of the subject is more active in the evening than the morning, and therefore to obtain satisfactory quantities of useful fHR data the scanning session must be longer in the morning than in the evening. As such, by taking contextual data into account, a more accurate remaining duration may be predicted.

In certain embodiments, analyzing the CTG data, the movement data and the artifacts may comprise providing the CTG data, the movement data and the artifacts to a machine learning algorithm, the machine learning algorithm being trained to predict the remaining duration of the CTG scanning session, and obtaining the predicted remaining duration from the machine learning algorithm.

The machine learning algorithm may be trained using a training algorithm configured to receive an array of training inputs and respective known outputs, wherein a training input comprises historical CTG data of a previous CTG scanning session of the subject, and wherein a respective known output comprises a historical CTG scanning duration of the previous CTG scanning session of the subject.

Thus, a machine learning algorithm may be able to identify patterns in the obtained data not typically noticeable by standard models and algorithms. Accordingly, a more accurate prediction of the remaining duration of the scanning session may be acquired by utilizing this tool.

In some embodiments, the CTG data may be obtained from at least one of an ultrasound Doppler based CTG sensor, or an electrophysiology based CTG sensor.

Furthermore, the movement data may be obtained from at least one of an accelerometer and a muscle electromyography (EMG) sensor.

In some embodiments, the method may also comprise determining a probability of session completion within at least one of a predetermined number of times based on the predicted remaining duration.

For example, the probability of finishing the session within 5 minutes, 10 minutes, 15 minutes, and so on may be determined from the predicted remaining duration. This information may be more useful for the subject being scanned to understand how long the scanning session may take. Indeed, providing probabilities (rather than a supply of only an anticipated end time) may be more useful for planning purposes.

In other embodiments, the method may further comprise generating, based on the analysis, a recommendation describing an action to be taken to reduce the remaining duration.

In this way, an overall remaining session duration required to acquire adequate CTG data may be shortened. For example, by advising the subject to stay still in response to detection of many movement-related artifacts, the quality of CTG data being acquired may be improved. This may be particularly useful when the CTG scanning session is performed at home by an untrained individual. In this case, the knowledge of a health care professional may not be forthcoming, and therefore any advice that can be provided may be useful for improving the scanning session.

According to other examples in accordance with an aspect of the invention there is provided a computer program comprising computer program code means adapted, when said computer program is run on a computer, to implement a method of predicting a remaining duration of a CTG scanning session.

According to additional examples in accordance with an aspect of the invention there is provided a system for predicting a remaining duration of a cardiotocography, CTG, scanning session, the system comprising: an interface configured to obtain CTG data associated with a subject during a CTG scanning session, and movement data describing a movement of the subject during the CTG scanning session; and a processor configured to: identify artifacts in the CTG data associated with the movement of the subject based on the CTG data and the movement data; analyze the CTG data, the movement data and the artifacts to predict a remaining duration of the CTG scanning session.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 presents a flow diagram of a method of predicting a remaining duration of a cardiotocography (CTG) scanning session of a subject according to an embodiment of the invention;
Fig. 2 presents a flow diagram of a method for matching the subject to other similar subjects according to an aspect of an embodiment of the invention;

Fig. 3 presents a system for predicting a remaining duration of a CTG scanning session of a subject according to a further embodiment of the invention; and

Fig. 4 is a simplified block diagram of a computer within which one or more parts of an embodiment may be employed.

DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should also be understood that the Figures are merely schematic and are not drawn to scale. It should also be understood that the same reference numerals are used throughout the Figures to indicate the same or similar parts.

The invention proposes concepts for predicting the remaining duration of a cardiotocography (CTG) scanning session of a subject. In particular, it is proposed to leverage CTG data and movement data from the scanning session to identify artifacts in the CTG data, and then use the CTG, movement and artifact data to produce a predicted remaining duration of the CTG scanning session. In this way, a total remaining time may be automatically assessed without the need for a skilled healthcare professional, enabling accurate assessment of required remaining duration of the CTG scanning to acquire sufficient data for diagnostic purposes. Indeed, such concepts may be particularly useful for remote (i.e. at home) CTG scanning sessions.

By way of explanation, dependent on the progress of the CTG measurement session (i.e. the signal coverage percentage), in view of the signal dropouts, the remaining duration of a CTG scanning session can vary. Put another way, due to varying levels of signal dropout and signal coverage, a total duration of a scanning session required for adequate signal acquisition varies. Currently, there are no tools available to facilitate prediction/estimation of a total duration of a measurement/scanning session required to produce sufficient usable data for diagnostic decisions during progression of the scanning session. This estimation is currently performed by a skilled health professional conducting the CTG scanning session.

Furthermore, it is envisioned that the future developments of electrophysiology-based CTG (eCTG), will improve remote (e.g. at home) fetal monitoring solutions. When conducting the CTG at home, a pregnant patient, or other untrained user, performs the measurements with a sensing device. For these non-professional users, it is even more important to facilitate a determination of a total (remaining) duration of the measurement session that would produce usable data for diagnostic decision making.

Thus, embodiments of the proposed invention seek to improve the comfort of a user conducting a CTG scanning session providing them with an automatic estimation of the CTG measurement session duration/remaining duration.

Although the need for these type of solutions is even higher for eCTG sensing, the solution would as well be relevant for traditional ultrasound Doppler based CTG.

More particularly, recently CTG monitoring devices have been provided that are based on electrophysiology-based CTG (eCTG.) These eCTG solutions may provide a higher resolution of fetal heart rate information. Specifically, eCTG may provide peak-to-peak fHR, whereas ultrasound Doppler detection may simply provide an average fHR over several heartbeats. This may enable the derivation of fHR variability and even a fetal ECG waveform (i.e. clinically relevant information that is currently not available from ultrasound Doppler detection).

Further still, eCTG has been shown to work better for those with high BMI, and to be more comfortable for pregnant mothers. Conducting an eCTG has also showed to be less burdensome for health care professionals, since it does not require frequent repositioning as is the case for ultrasound Doppler based CTG fetal monitors.

Frequent necessary visits to the hospital or hospitalization for the purpose of monitoring (including CTG) presents a large burden for those who are pregnant. A means for reducing this burden is the use of a travelling hospital midwife to conduct a CTG scanning session at a patient's home. However, this is particularly labor intensive. Therefore, remote monitoring solutions have been developed to conduct the measurements at home by the patients themselves, with the data resultant data transferred to a healthcare professional for review.

Since eCTG technology is new, enabling remote fetal monitoring based on eCTG technology presents new challenges.

Specifically, eCTG measurements are conducted in a different way than ultrasound Doppler CTG. Typically, a sensing ECG patch with 5-6 adhesive electrodes is attached to the belly of a pregnant patient. After the impedance indicators for all the electrodes show positive feedback on a fetal monitor, the measurements are conducted by leaving the patch on the patient for 30 to 60 min. In contrast, ultrasound Doppler based CTG solutions typically involve using two round transducers, one having piezoelectric elements used for measuring the fHR and another having a strain gauge for measuring maternal contractions. These transducers are placed on the belly of a pregnant patient in order to locate the fetal heart using auditory feedback. The transducers are then fixed in place by wrapping and tightening elastic belts around the belly.

In case of the ultrasound Doppler CTG, if the fHR signal gets lost (e.g. due to the fetus changing its position), the fetal monitor produces an auditory alarm. This will notify the healthcare professional conducting the scan to reposition the transducer in order to continue the measurements. In standard practice, the healthcare professional assesses the acquired measurements of the fHR and UA (i.e. via a print-out), accounts for the number of times the fHR was lost and repositioning was required, and determines the additional measuring time required.

In contrast, when acquiring the fHR signal with eCTG, the electrodes of the device pick up an electric signal. This signal corresponds to a mixture of different bioelectric signals generated by the beating of the fetal heart, maternal heart, contracting uterus and other physiological processes (muscle activity and motion artifacts). Individual signals related to the maternal heart rate (mHR), fHR and UA are extracted by processing the raw compound signal (i.e. applying a blind source separation technique). Extracting the fHR is particularly difficult due to the fHR signal amplitude being significantly (~5 times) smaller than that of the mHR, meaning a poor signal-to-noise ratio. In particular, at moments when the noise level becomes relatively high (i.e. due to movement of the pregnant patient, high levels of UA, etc.), the fHR signal may not be accurately detected. In other words, signal dropouts for the fHR may be common. Thus, detection of the fHR using eCTG during the second stage of labor is typically very challenging. When this occurs and there are no simple remedies (such as the reattachment of loose electrodes), the only option is to wait until fHR detection is restored.

Thus, it is desirable to provide a means for automatically determining/prediction/estimating a remaining duration of a scanning session required, such that sufficient data for diagnostic purposes may be acquired during the scanning session.

According to a specific exemplary embodiment, the following elements may be provided:

(i) A sensing device, such as CTG transducers or an eCTG sensing patch, for signal acquisition;
(ii) Additional sensors, such as an accelerometer, or additional ECG sensors. These can be used for detecting maternal movements by acceleration or muscle electromyography (EMG) that could be a source of motion artifacts;
(iii) A processing unit for:

(a) extracting different features related to the signals of interest. Signals of interest may include a fHR, a mHR, and UA acceleration and muscle contractions. Features of such signals include a duration of uninterrupted fHR measurements, a presence or absence of UA signal, etc.;
(b) calculating, based on an algorithm/model, a predicted total (remaining) duration of the CTG scanning session. This may take into account the fHR signal, mHR signal, UA signal, additional sensor signals (i.e. maternal movement signals), and the extracted features;

(iv) A storage for model instructions and ECG, movement and other data related to the current scanning session, as well as other relevant data (i.e. contextual information, physiological data, and previous ECG data;
(v) A user interface for displaying the predicted total (remaining) duration of the measuring session. The user interface may further provide a measurement progress (i.e. a percentage or progress bar), an estimated end-time, and other forms of feedback to the user. The user interface may also provide information advising the pregnant patient to be still in case their movements lead to too many fHR dropouts;
(vi) A means/system for a healthcare professional (who reviews the CTG data from a remote location), to make manual adjustments to the predicted remaining duration of the scanning session;
(vii) A feedback interface to collect feedback from the user, such as information regarding the estimate accuracy.

At least some of the system components described above could be part of a fetal monitor. Thus, the processing unit for data analysis could be realized on the fetal monitor CPU. Alternatively, at least some of the system components could be provided on a personal device, such as a tablet that has both the screen for the user interface, and a CPU for data processing.

Moreover, embodiments of the invention may provide that uterine activity (UA) is detected. Contractions (detected based on uterine activity) provide additional useful data, and may help to analyze how the fetus reacts to the stress of the contraction by analysing fHR decelerations relative to the contractions on and off sets. Moreover, contractions data is a useful measurement for Preterm Labor risk patients. The model could be programmed to extend the measurements session duration in case of detecting UA. For example, after detecting UA the measurements time could be extended to additional 20 minutes.

In another embodiment, the predicted remaining duration may be dependent on the sleep-wake state of the fetus, since CTG can typically be interpreted when a fetus is not in a deep sleep state. Usually, this determination is made by a healthcare professional who asks the pregnant patient whether they feel any fetal movement, and how frequent the fetal movement is. In the case of the present invention, this may be detected by means of fetal movement detection, in the form of movement data.

In further embodiments, after accumulating a substantial amount of data from different subjects, subjects may be clustered to identify groups/classes of subjects that are similar according to their physiological characteristics (i.e. demographics, obstetric history, etc.). The average scanning session time for the corresponding cluster with the highest similarity to the subject being scanned may then be leveraged to determine the predicted remaining duration of the measurement session.

In additional embodiments, previous CTG data recorded for the subject being scanned may be used. This data could be integrated with extracted features by a machine learning model in order to predict the total/remaining scanning session duration. Further, feedback from a user (i.e. a healthcare professional or pregnant patient) regarding the accuracy of the estimate may be used for training and improvements of duration prediction.

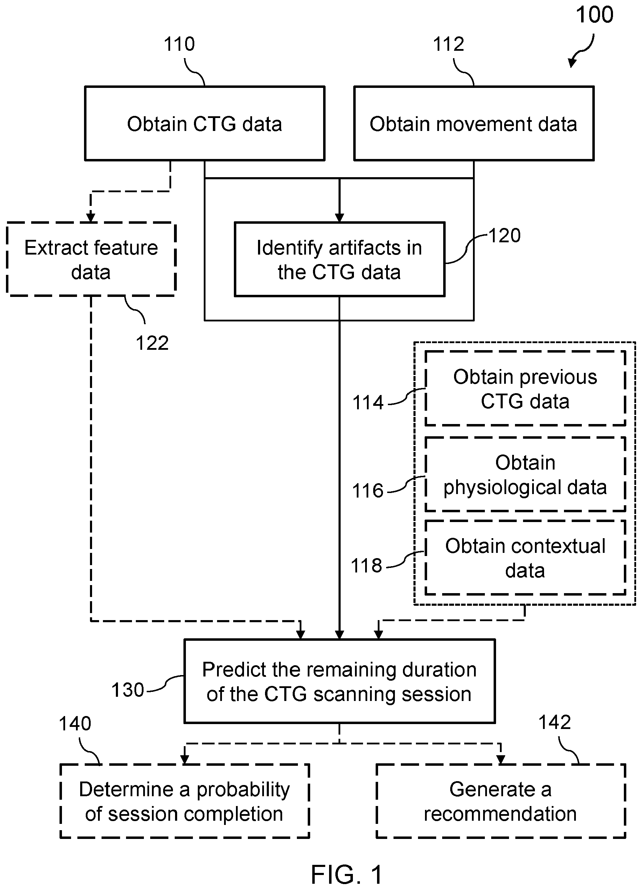

Moving onto Fig. 1, there is provided a flow diagram of a method of predicting a remaining duration of a CTG scanning session of a subject. In other words, the method is used for predicting an amount of remaining time of a CTG scanning session required to gather adequate CTG data of the subject.

To clarify, the subject in this context may refer to either the pregnant subject, the fetus of the pregnant subject, or both. Indeed, the CTG scanning session is necessarily performed on the pregnant subject, but is usually with the aim of measuring the fHR.

Prediction of the remaining duration may be performed just once, continually from beginning to the end of the scanning session, or selectively (i.e. when a CTG signal drop-out is detected, or at predetermined intervals). Indeed, prediction of the remaining duration may be performed by predicting the total required duration, and subtracting the already performed time. The predicted remaining duration may also be performed by adjusting (i.e. increasing/decreasing) a target end time, based on the following.

At step 110, CTG data associated with the subject during a CTG scanning session is obtained. The CTG data may be obtained/acquired from an ultrasound Doppler based CTG sensor, and/or an electrophysiology based CTG sensor. Indeed, any device appropriate for performing CTG may be used.

The CTG data may comprise an fHR signal, a maternal heart rate (mHR) signal, and a uterine activity (UA) signal. Such signals may not be completely distinct, but may be separated by techniques known to the person skilled in the art. Furthermore, due to the nature of CTG scanning and the use of such devices, there may also be noise present in the CTG data. However, this may be removed/suppressed/minimized by pre-processing for reducing noise, such as via an appropriate filter.

At step 112, movement data describing a movement of the subject during the CTG scanning session is obtained. In particular, the movement data may relate to either the pregnant subject themselves (i.e. repositioning, accidental jolting, etc.) and/or may relate to movement of the fetus.

The movement data may be obtained/acquired from an accelerometer and/or a muscle electromyography (EMG) sensor. Indeed, any sensor capable of detecting movement of either or both of the pregnant patient and the fetus may be utilised, as would be appreciated by the skilled person.

At step 120, artifacts in the CTG data associated with the movement of the subject are identified. The identification is based on the CTG data and the movement data.

In other words, the CTG data is analyzed in the context of the movement data in order to identify drop-outs in the CTG signal associated with the movement data. Such drop-outs mean that the quality of the CTG data at these times may be of a reduced quality, such that the CTG data may not be useful (or of reduced usefulness) for diagnostic purposes. Thus, the identification of such artifacts is important for the accurate adjustment of the predicted remaining duration of the scanning session.

More specifically, movement of the fetus is relevant as an indicator of whether the fetus is in deep sleep state or not, which may be clinically relevant information for interpreting the CTG data in order to identify artifacts. Maternal movement is relevant since in case the pregnant subject moves a lot, there arises difficulties in extracting an fHR signal from the CTG data due to the presence of noise artifacts. In this case it may be pertinent to instruct the pregnant subject to reduce movement during the measurements, and to potentially increase the predicted remaining duration to adjust for these movements. Thus, movement of both the pregnant patient and the fetus during the scanning session may be useful for predicting the total required remaining duration of the scanning session by identifying artifacts in the CTG data.

At step 130, the CTG data, the movement data and the artifacts are analyzed in order to predict a remaining duration of the CTG scanning session. The data may be analyzed using an algorithm configured to predict the remaining duration responsive to inputting the data. For example, the algorithm may be a statistical model (i.e. a Bayesian model) or a machine-learning based model.

Indeed, it has been outlined previously how the CTG, movement and artifact data may be useful for indicating an amount of time remaining in the scanning session to ensure sufficient data useful for diagnosis. For example, a predetermined/average time may be adjusted on the basis of the analysis of the CTG, movement and artifact data in order to establish a remaining scanning session duration. Indeed, if there exist many artifacts within the CTG data, then a remaining scanning session time may be increased.

In some cases, analyzing the CTG data, the movement data and the artifacts comprises providing the CTG data, the movement data and the artifacts to a machine learning algorithm, the machine learning algorithm being trained to predict the remaining duration of the CTG scanning session, and obtaining the predicted remaining duration from the machine learning algorithm. The machine learning algorithm may be any machine learning, artificial intelligence and/or neural network-based algorithm that can learn from previous data to establish patterns in unseen data.

In particular, the machine learning algorithm may be trained using a training algorithm configured to receive an array of training inputs and respective known outputs, wherein a training input comprises historical CTG data of a previous CTG scanning session of the subject, and wherein a respective known output comprises a historical CTG scanning duration of the previous CTG scanning session of the subject.

Thus, historical data may be leveraged in order to establish a context for the obtained CTG and movement data, and therefore provide an accurate predicted remaining duration.

One specific example method for analyzing the CTG data is as follows. In order to leverage the CTG information to predict a remaining duration of the CTG scanning session, the signal loss registered since the start of the session may be determined. For CTG interpretation, a signal loss of less than 20% (i.e. signal being dropped less than 20% of the time) is considered to be acceptable according to agreed guidelines. If from the start of the scanning session, the signal loss starts exceeding this threshold, the predicted remaining duration of the scanning session may be increased, (which typically lasts ~30-60 minutes). Accordingly, this may compensate for the excessive signal loss and to meet the interpretability criteria.

A current percentage of the signal loss is x (i.e. percentage of obtained signal having a lost signal), and the additional percentage of the total scanning duration required to acquire adequate CTG data is y. Given this, and when x exceeds 20%, the following is true:

$$\frac{x}{(100 + y)} = 0.2$$

Of course, this can be written as:

$$y = 5x - 100$$

This calculation will lead to a total measuring time (when applied to the initial total scanning duration) provided there is no additional signal loss during the rest of the scanning session. In the case that further signal loss (represented by z) is detected, the additional scanning session duration will increase according to:

$$y = 5(x + z) - 100$$

Furthermore, movement data describing maternal movement (i.e. a signal from a sensor such as an accelerometer) is used. Indeed, maternal movement may lead to a signal loss percentage greater than 20%. In such a case, a recommendation may be made to the pregnant patient, and could be displayed on a user interface suggesting remaining as still as possible in order to improve signal coverage.

Optionally, the method may comprise one or more of the steps provided within the dotted outlines (steps 114, 116, 118, 122 140 and 142).

At step 122, the CTG data is processed to extract feature data that is indicative of a usability of the CTG data for diagnosis. In this case, predicting the remaining duration is further based on the extracted feature data. In other words, the CTG data is processed in order to determine a usability of the CTG data for diagnosis, by identifying/extracting/determining feature data from the CTG data.

Feature data may include any data that is useful for diagnosing the subject. For example, the feature data comprises at least one of a fetal heart rate measurement, a uterine activity measurement, and a fetal sleep status. More specifically, the fetal heart rate measurement may comprise heart rate features including a fetal heart rate variability, a fetal heart rate baseline, fetal heart rate accelerations, and fetal heart rate decelerations.

Thus, if it is determined that a fHR variability, fHR baseline and fHR acceleration and decelerations are derived from the CTG data, it may be determined that only a short amount of session duration remains. Indeed, if there is little feature data (i.e. not much data within the CTG data that may be useful for diagnosis), then the remaining scanning session time/duration may be increased.

At step 114, previous CTG data associated with/measured from the subject during at least one previous CTG scanning session is obtained. The prediction of the remaining duration is then further based on the previous CTG data.

In this way, feedback regarding previous/past/historic scanning session of the subject may be taken into account. The at least one previous scanning session may comprise not only multiple scanning sessions from the pregnant subject's current pregnancy, but also previous pregnancies. The CTG data of these previous scanning sessions may be obtained from a database of previous CTG scanning sessions. Said CTG data may also comprise metadata comprising information regarding a context of the scan (i.e. a time of day, a gestational age, an acquisition device etc.) that may provide information useful for assessing a relevance of the CTG data.

At step 116, physiological data describing one or more characteristics of the subject is obtained. Predicting the remaining duration is then further based on the physiological data. Physiological data is any data describing physical characteristics of the subject (i.e. demographics, past medical conditions, etc.), each of which may indicate an amount of remaining time required for satisfactory CTG data acquisition.

The physiological data may be obtained from a user, or from a database(s) having information stored about the pregnant subject.

For example, the physiological data comprises at least one of an age, a gestational age, a height, a weight, medical conditions and a medical history. However, the skilled person would appreciate additional physiological information that may provide information regarding the likely quality of the CTG data, and thus the predicted remaining duration of the scanning session.

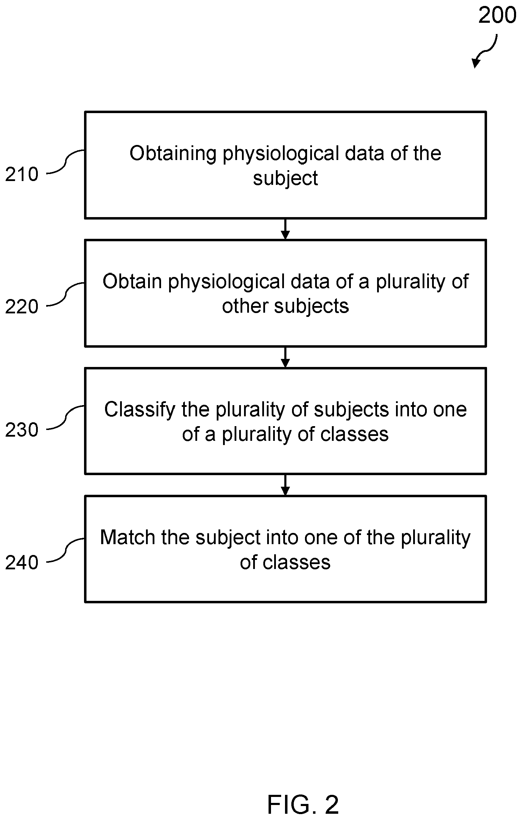

Furthermore, the use of the obtained physiological data will be explained in more detail in reference to Fig. 2 below.

At step 118, contextual information describing a context of the CTG scanning session is obtained. Thus, the prediction of the remaining duration is further based on the contextual information. Any contextual information (i.e. data about the surrounding environment, and conditions of the scanning session) may provide clues as to the length of the scanning session required for satisfactory CTG data acquisition.

Such data may be manually input by a user, or may be obtained from additional sensors.

For example, the contextual information comprises at least one of a time of day, a location, a device used to obtain the CTG data, and an ambient temperature. However, the skilled person would appreciate additional contextual data that may provide information regarding the likely quality of the CTG data, and thus the predicted remaining duration of the scanning session.

Of course, the information obtained in steps 114-118 may also be utilized by a machine learning algorithm (and historical data used to train the machine learning algorithm), which is configured to predict a remaining duration of the scanning session (i.e. similar to the CTG, movement and artifact data described above). Additionally and/or alternatively, said data may be used to identify artifacts in the CTG data, and also may be used in the extraction of feature data as described above.

Finally, steps 140 and 142 present steps wherein the predicted remaining duration and analysis of the above data of the scanning session is used to generate outputs useful for the subject, a user, and/or a healthcare professional.

At step 140, a probability of session completion within at least one of a predetermined number of times is determined based on the predicted remaining duration. For example, the probability of finishing the session within 5 minutes, 10 minutes, 15 minutes, and so on may be determined from the predicted remaining duration.

At step 142, a recommendation describing an action to be taken to reduce the remaining duration is generated based on the analysis of the CTG, movement and artifact data. For example, a recommendation to remain still during the examination, to conduct the scanning session at an alternative time, or to reposition the CTG sensor may be provided.

Indeed, the data generated in steps 140 and 142 may subsequently be output to a user. Alternative and/or additional outputs may also be considered, including simply displaying the predicted remaining duration.

Fig. 2 presents a flow diagram of a method for matching the subject to other similar subjects based on the physiological data obtained in step 116 of the method described in relation to Fig. 1.

Indeed, matching the subject with a plurality of similar subjects may be based on the physiological data of the subject and physiological data of the plurality of other subjects, wherein each of the plurality of similar subjects are associated with historical CTG scanning session duration data. In this case, predicted remaining duration is further based on the historical CTG scanning session duration data.

In a simple example, when the CTG scanning session duration data of a plurality of other (similar) subjects is known, then this duration data may be used to inform the prediction of the remaining session duration. Similar subjects may often experience roughly similar scanning session durations, and therefore taking this into account may help to provide an accurate prediction.

Specifically, steps 210-240 provide a method of matching the subject with the plurality of other subjects.

At 210, physiological data of the subject is obtained. This is the same as the step 116 described in reference to Fig. 1, and therefore further explanation is omitted here for the sake of brevity.

At step 220, physiological data of each of the plurality of other subjects is obtained. This may be obtained from a large database of other subjects and their associated CTG data. The physiological data of the other subjects may relate to characteristics that are the same or similar to the characteristics described by the physiological data of the subject (i.e. relate to age, gestational age, a weight, etc.).

At step 230, the plurality of other subjects are clustered based on the physiological data such that similar subjects are classified into one of a plurality of classes. The clustering may be performed by any method known to the skilled person for clustering subjects based on values relating to the same parameter.

At step 240, the subject is matched to one of the plurality of classes based on the physiological data of the subject. This means that the class having subjects having the most similar physiological characteristics is identified, and the data of the subjects in that class may be used for the prediction of the remaining duration of the scanning session.

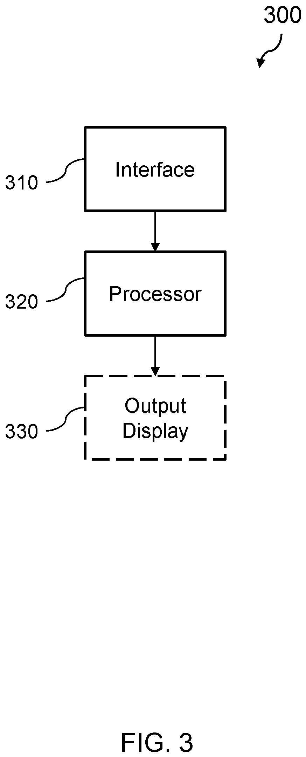

Fig. 3 presents a system 300 for predicting a remaining duration of a CTG scanning session of a subject. In particular, the system comprises an interface 310 and a processor 310, and may optionally further comprise an output display 330. Of course, each of the interface 310, processor 320 and output display 330 may comprise sub-components for handling various functions of each module. The interface 310, processor 320 and output display 330 may be provided by one integrated device (i.e. a smartphone/tablet).

Firstly, the interface 310 is at least configured to obtain CTG data associated with a subject during a CTG scanning session, and movement data describing a movement of the subject during the CTG scanning session. Depending on the embodiment, the interface 310 may also obtain physiological data of the subject, physiological data of other subjects, contextual data of the scanning session, and previous CTG data of previous scanning sessions of the subject. The interface 310 may store such data locally, or may acquire such information from one or more storages/databases of such information.

Moreover, the interface 310 then may pre-process this information, or may simply pass this information to the processor.

The processor 320 is configured to identify artifacts in the CTG data associated with the movement of the subject based on the CTG data and the movement data. Indeed, the processor 320 may also take into account physiological data of the subject, physiological data of other subjects, contextual data of the scanning session, and previous CTG data of previous scanning sessions of the subject to identify the artifacts.

Furthermore, the processor 320 is configured to analyze the CTG data, the movement data and the artifacts to predict a remaining duration of the CTG scanning session. Also, the processor 320 may additionally take into account physiological data of the subject, physiological data of other subjects, contextual data of the scanning session, and previous CTG data of previous scanning sessions of the subject to identify the artifacts.

The processor 320 may then generate a probability of session completion within at least one of a predetermined number of times is determined based on the predicted remaining duration. Also, the processor 320 may generate a recommendation describing an action to be taken to reduce the remaining duration generated based on the analysis of the CTG, movement and artifact data (and other data, if obtained).

The predicted remaining duration of the scanning session may be provided to the output display 330, which may provide this (and other generated data) to a user (i.e. the pregnant patient, a healthcare professional and/or a person conducting the scanning session).

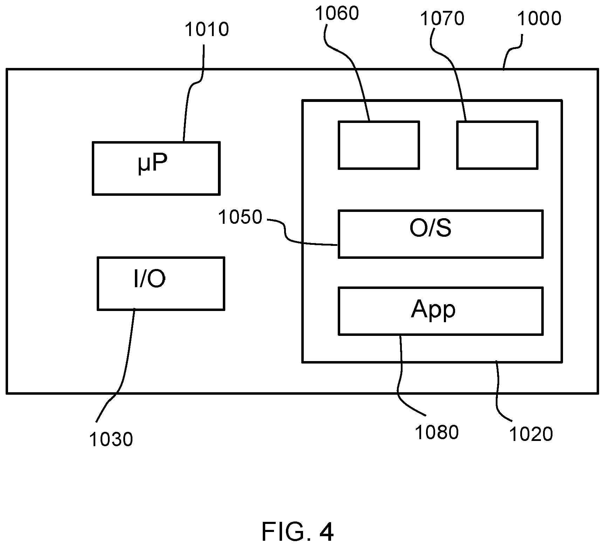

Fig. 4 illustrates an example of a computer 1000 within which one or more parts of an embodiment may be employed. Various operations discussed above may utilize the capabilities of the computer 1000. For example, one or more parts of a system for predicting a remaining duration of a CTG scanning session of a subject according to another embodiment of the invention may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 1 and 2, and the system(s) described in relation to Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and nonvolatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A method (100) for predicting a remaining duration of a cardiotocography, CTG, scanning session of a subject, the method comprising:

    obtaining (110) CTG data associated with the subject during a CTG scanning session;
    obtaining (112) movement data describing a movement of the subject during the CTG scanning session;
    identifying (120) artifacts in the CTG data associated with the movement of the subject based on the CTG data and the movement data; and
    analyzing (130) the CTG data, the movement data and the artifacts to predict a remaining duration of the CTG scanning session.

2. The method of claim 1, further comprising processing (122) the CTG data to extract feature data indicative of a usability of the CTG data for diagnosis, wherein predicting the remaining duration is further based on the extracted feature data.

3. The method of claim 2, wherein the feature data comprises at least one of a fetal heart rate measurement, a uterine activity measurement, and a fetal wake status, and optionally wherein the fetal heart rate measurement comprises heart rate features including a fetal heart rate variability, a fetal baseline heart rate, fetal heart rate accelerations, and fetal heart rate decelerations.

4. The method of any of claims 1-3, further comprising obtaining (114) previous CTG data associated with the subject during at least one previous CTG scanning session, and wherein predicting the remaining duration is further based on the previous CTG data.

5. The method of any of claims 1-4, further comprising obtaining (116) physiological data describing one or more characteristics of the subject, and wherein predicting the remaining duration is further based on the physiological data, and optionally wherein the physiological data comprises at least one of an age, a gestational age, a height, a weight, medical conditions and a medical history.

6. The method of claim 5, further comprising:

    matching (200) the subject with a plurality of similar subjects based on the physiological data of the subject and physiological data of the plurality of other subjects, wherein each of the plurality of similar subjects are associated with historical CTG scanning session duration data; and wherein the predicted remaining duration is further based on the historical CTG scanning session duration data.

7. The method of claim 6, wherein matching (200) the subject comprises:

obtaining (220) physiological data of each of the plurality of other subjects;

clustering (230) the plurality of other subjects based on the physiological data to classify similar subjects into one of a plurality of classes; and

matching (240) the subject to one of the plurality of classes based on the physiological data of the subject.

8. The method of any of claims 1-7, further comprising obtaining (118) contextual information describing a context of the CTG scanning session, and wherein predicting the remaining duration is further based on the contextual information, and optionally wherein the contextual information comprises at least one of a time of day, a location, an ambient temperature and a device used to obtain the CTG data.

9. The method of any of claims 1-8, wherein analyzing the CTG data, the movement data and the artifacts comprises:

providing the CTG data, the movement data and the artifacts to a machine learning algorithm, the machine learning algorithm being trained to predict the remaining duration of the CTG scanning session; and

obtaining the predicted remaining duration from the machine learning algorithm.

10. The method of claim 9, wherein the machine learning algorithm is trained using a training algorithm configured to receive an array of training inputs and respective known outputs, wherein a training input comprises historical CTG data of a previous CTG scanning session of the subject, and wherein a respective known output comprises a historical CTG scanning duration of the previous CTG scanning session of the subject.

11. The method of any of claims 1-10, wherein the CTG data is obtained from at least one of an ultrasound Doppler based CTG sensor, or an electrophysiology based CTG sensor, and wherein the movement data is obtained from at least one of an accelerometer and a muscle electromyography, EMG, sensor.

12. The method of any of claims 1-11, further comprising determining (140) a probability of session completion within at least one of a predetermined number of times based on the predicted remaining duration.

13. The method of any of claims 1-12, further comprising generating (142), based on the analysis, a recommendation describing an action to be taken to reduce the remaining duration.

14. A computer program comprising computer program code means adapted, when said computer program is run on a computer, to implement the method of any of claims 1-13.

15. A system (300) for predicting a remaining duration of a cardiotocography, CTG, scanning session, the system comprising:

an interface (310) configured to obtain CTG data associated with a subject during a CTG scanning session, and movement data describing a movement of the subject during the CTG scanning session; and

a processor (320) configured to:

identify artifacts in the CTG data associated with the movement of the subject based on the CTG data and the movement data;

analyze the CTG data, the movement data and the artifacts to predict a remaining duration of the CTG scanning session.

100

110

Obtain CTG data

112

Obtain movement data

Extract feature
data

122

Identify artifacts in
the CTG data

120

Obtain previous
CTG data

114

Obtain
physiological data

116

Obtain contextual
data

118

Predict the remaining duration
of the CTG scanning session

130

140

Determine a probability
of session completion

142

Generate a
recommendation

FIG. 1

200

210 — Obtaining physiological data of the subject

220 — Obtain physiological data of a plurality of other subjects

230 — Classify the plurality of subjects into one of a plurality of classes

240 — Match the subject into one of the plurality of classes

FIG. 2

300

FIG. 3

FIG. 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/192027 A1 (WOHLSCHLAGER MARKUS [DE] ET AL) 27 June 2019 (2019-06-27) * paragraphs [0001], [0012] – [0057], [0072] – [0116] * | 1-15 | INV. A61B8/08 A61B8/00 |
| A | US 2010/185108 A1 (VULLINGS RIK [NL] ET AL) 22 July 2010 (2010-07-22) * paragraphs [0001], [0005] – [0045], [0056] – [0063]; claims; figures * | 1-15 | |
| A | US 2020/085365 A1 (MCDONALD SARAH CATHERINE [AU] ET AL) 19 March 2020 (2020-03-19) * paragraphs [0007] – [0065]; claims; figures * | 1-15 | |

TECHNICAL FIELDS
SEARCHED       (IPC)

A61B

The present search report has been drawn up for all claims

| Place of search | Date of completion of the search | Examiner |
|---|---|---|
| Munich | 3 April 2023 | Mundakapadam, S |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
....................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 20 6568

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2023

| Patent document cited in search report | | Publication date | Patent family member(s) | | Publication date |
|---|---|---|---|---|---|
| US 2019192027 | A1 | 27-06-2019 | CN | 109561839 A | 02-04-2019 |
| | | | EP | 3496602 A1 | 19-06-2019 |
| | | | US | 2019192027 A1 | 27-06-2019 |
| | | | WO | 2018029120 A1 | 15-02-2018 |
| US 2010185108 | A1 | 22-07-2010 | DK | 2185068 T3 | 02-01-2017 |
| | | | EP | 2185068 A1 | 19-05-2010 |
| | | | ES | 2606216 T3 | 23-03-2017 |
| | | | PL | 2185068 T3 | 30-06-2017 |
| | | | US | 2010185108 A1 | 22-07-2010 |
| | | | WO | 2009013246 A1 | 29-01-2009 |
| US 2020085365 | A1 | 19-03-2020 | AU | 2017371395 A1 | 20-06-2019 |
| | | | BR | 112019011959 A2 | 05-11-2019 |
| | | | CA | 3046352 A1 | 14-06-2018 |
| | | | CN | 110290742 A | 27-09-2019 |
| | | | EP | 3551058 A1 | 16-10-2019 |
| | | | JP | 7181201 B2 | 30-11-2022 |
| | | | JP | 2020513277 A | 14-05-2020 |
| | | | JP | 2023017993 A | 07-02-2023 |
| | | | KR | 20190101397 A | 30-08-2019 |
| | | | NZ | 754277 A | 26-11-2021 |
| | | | US | 2020085365 A1 | 19-03-2020 |
| | | | US | 2022167911 A1 | 02-06-2022 |
| | | | WO | 2018102874 A1 | 14-06-2018 |

EPO FORM P0459

For more details about this annex : see Official Journal of the European Patent Office, No. 12/82